(19) Europäisches Patentamt / European Patent Office / Office européen des brevets

(11) **EP 2 815 778 B1**

(12) **EUROPEAN PATENT SPECIFICATION**

(45) Date of publication and mention
of the grant of the patent:
**16.08.2017 Bulletin 2017/33**

(51) Int Cl.:
*A61M 5/14* (2006.01)      *A61M 5/142* (2006.01)
*A61M 5/168* (2006.01)      *A61M 5/50* (2006.01)
*A61M 5/178* (2006.01)      *A61M 5/20* (2006.01)
*A61M 5/31* (2006.01)

(21) Application number: **13173246.3**

(22) Date of filing: **21.06.2013**

(54) **Portable infusion device for infants with refillable small volume dosing unit**

Tragbares Infusionsgerät für Kinder mit wiederbefüllbarer Dosiereinheit kleinen Volumeninhalts

Dispositif de perfusion portable pour enfants avec unité de dosage remplissable de capacité faible

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**24.12.2014 Bulletin 2014/52**

(73) Proprietors:
• **F. Hoffmann-La Roche AG**
**4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Roche Diabetes Care GmbH**
**68305 Mannheim (DE)**
Designated Contracting States:
**DE**

(72) Inventor: **Geipel, Andreas**
**68542 Heddesheim (DE)**

(74) Representative: **Rentsch Partner AG**
**Fraumünsterstrasse 9**
**Postfach**
**8022 Zürich (CH)**

(56) References cited:
**EP-A1- 1 754 505         EP-A1- 1 970 677**
**EP-A1- 2 163 273         EP-A1- 2 457 602**
**EP-A1- 2 510 960         WO-A1-2009/065801**
**WO-A1-2010/008575       WO-A1-2013/034159**
**WO-A2-2011/097487       DE-A1- 3 515 624**
**US-A- 3 714 943          US-A1- 2008 051 709**
**US-A1- 2010 049 127      US-A1- 2011 224 644**
**US-A1- 2011 300 001      US-B2- 7 942 863**

## Description

### Technical field

The present disclosure is directed towards dosing units for ambulatory infusion systems and towards ambulatory infusion systems including a dosing unit. The disclosure is further directed towards storage boxes for drug containers, and towards a method for storing a drug container. The storage boxes may especially used in combination with or as part of an ambulatory infusion system in accordance with the present disclosure.

### Background

Continuous Subcutaneous Insulin Infusion (CSII) is a well-established form of state-of-the-art therapy of diabetes mellitus. In CSII, a diabetic patient carries a miniaturized insulin pump substantially continuously, night and day, and receives substantially continuous infusions of a liquid insulin formulation into the subcutaneous tissue according to a typically time-variable basal infusion schedule that meets his or her continuous insulin demand. In addition, typical insulin pumps may infuse larger drug boli on demand to meet the patient's increased insulin demand in the context of carbohydrate intake and under certain exceptional circumstances. The insulin is typically infused via a subcutaneous cannula that is coupled to the insulin pump via tubing and is exchanged - together with the tubing - by a user, i.e., the patient or another person such as a partner, parent, etc., every few days. Alternatively, the insulin pump may be directly attached to the patient's body in form of a patch. In this case, the insulin pump is fully or partly disposable. A drug container that is comprised in a container compartment of typical insulin pumps holds the required insulin amount for a number of days and is, when empty, replaced by a new container. The container may be available readily filled, e.g. in form of largely available Pentype cartridges, or may be a special-purpose container that is filled by the user prior to use.

In the framework of CSII, the therapy of diabetic children and in particular of newborns and infants up to an age of some years- in the following generally referred to as "infants" - is especially critical. A particular challenge in this context is given by the fact that a diabetic's total daily insulin demand, the TDD increases with the body weight and is accordingly low for infants. With insulin doses being typically measured in International Units (IU) as standard measure for substances based on their biological effect, and the body weight being measured in kg, a known rule-of-thumb for the TDD is given by

$$TDD \approx 0.5 \ IU \ / \ kg$$

That is, the TDD for an infant having a body weight of 10 kg is about 5 IU, and for an infant of 20 kg (corresponding to a typical age of 4 years) is about 10 IU.

The drug containers of typical available insulin pumps have a filling volume of e.g., 3 ml, corresponding to 300 IU of liquid insulin formulation for the most common concentration U100 (100 IU per ml of liquid). For the above-given examples, this corresponds to the 30fold TDD of a infant with 20 kg and to the 60-fold TDD of an infant with 10 kg. Since liquid insulin should be generally stored at low temperatures, it is not possible to maintain insulin at room temperature or even higher body temperature for such a period of time. At or above room temperature, the biological activity is inevitably reduced in a hardly predictable way over time.

In addition, all infusion pumps, are to some degree, susceptible to dosing errors, resulting, e.g., from thermal expansion and constriction of the liquid insulin as well as the reservoir and the pump, resulting from ambient temperature changes. Those dosing errors are known to occasionally result in severe and potentially dangerous medical complications even for adult patients. In a particularly hazardous scenario, the complete reservoir content is infused at once, e.g., because of a pump defect. For the above-given example of an infant having a body weight of 10 kg, this would - in the worst case - mean an infusion of the 60-fold daily dose, likely resulting in the infant's death.

A further problem with the therapy of infants by CSII is the limited dosing precision and resolution of current insulin pumps, according to the general device design and factors such as slackness, tolerances, and wear. Since the biological effect of a given volumetric amount of over- or under-dosing increases with the TDD decreasing, the dosing precision is typically by far sufficient for most adults while it is critically and potentially insufficient for infants. The same holds true for the smallest amounts that can be programmed for bolus infusion and as basal delivery rate.

To cope with the problems and challenges as described above, it is common in CSII therapy of infants to dilute the insulin with saline solution, thus reducing the "effective" insulin concentration. This approach, however, is cumbersome, time consuming and susceptible to potentially dangerous mistakes. In addition, this approach does not improve the situation with respect to the size of insulin pumps that is - though sufficiently miniaturized to allow convenient and discrete carrying, e.g., in a trousers pocket by most adults and older juveniles - generally unfavourably bulky for infants and may even adversely affect their natural development.

In summary, no satisfying solution is currently available for CSII therapy of infants. For medical reasons, however, this form of therapy is desirable.

### Summary of disclosure

The present disclosure aims at improving the situation concerning the CSII therapy of infants.

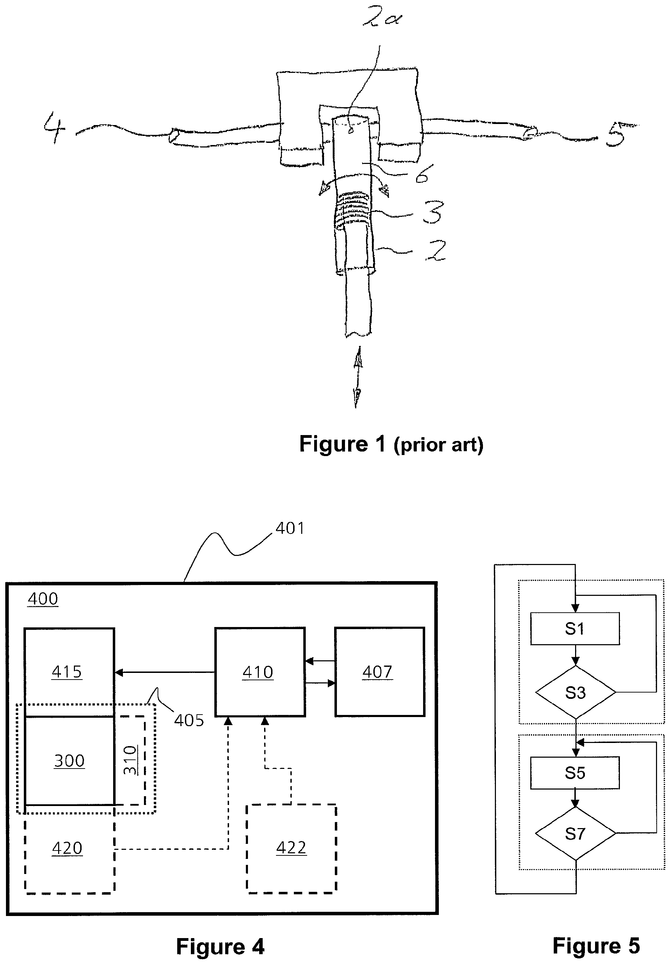

The EP 1970 677 A1 discloses an infusion system with a dosing unit as exemplarily shown in Figure 1. The dosing unit includes a pump cylinder (2) with a dosing volume (6) that may be varied in steps or increments by displacing a piston (3). The pump cylinder (2) may have a single opening (2a) for filling the variable volume with drug via a supply tube (4) from a drug container and expelling drug from the variable volume into a catheter or infusion line (5). By rotating the pump cylinder (2), together with the piston (3), about its longitudinal axis with respect to a sealing stationary member (not referenced, U-shaped piece in Figure 1), aperture (2a) in a wall of pump cylinder (2), and, thereby the dosing volume (6), may be alternatively fluidic connected with the supply tube (4) or with the catheter or infusion line (5) for infusion drug out of the dosing volume (6). The pump cylinder and the stationary member, in combination, form a control valve that controls liquid flow to and from the dosing volume.

In the exemplary design shown in Figure 1, piston (3) has, in the proximal end section, an outer thread that is in engagement with a (not shown) corresponding inner thread of pump cylinder 2. In that way, piston (3) may be displaced inside pump cylinder (2) in a screw-like way by providing a driving torque to a plunger shaft without moving the pump cylinder (2). By selectively coupling, e.g. via selective frictional coupling, cylinder (2) and piston (3), both cylinder (2) and piston (3) may be rotated together, that is, without relative motion relative to the stationary member for valve switching.

When operating such a dosing unit, dosing volume (6) is first filled by fluidic connecting it with the drug container while the connection to the catheter or infusion line (5) is selling closed, followed by retracting the piston (3), thus increasing the variable volume and drawing drug from the drug container into the variable volume via supply tube (4). Subsequently, the variable volume is fluidic connected with the catheter or infusion line while the connection to the drug container is sealing closed. The variable volume is emptied by decreasing the variable volume in incremental steps in accordance with the patient's basal and bolus insulin demand. When fully or substantially emptied, the dosing volume (6) is filled again from the drug container as described above. Since the maximum filling volume of the pump cylinder is considerably smaller than the total volume of the drug container, it allows volumetric dosing by displacing the piston of the dosing unit with considerably higher precision as compared to the drug container. At the same time, the dosing unit may be designed such that it can, in contrast e.g. to solid-state micro pumps, be manufactured using well-established and cost-efficient large-scale technology, in particular injection moulding.

Due to the relatively small filling volume of the pump cylinder, however, the pump cylinder needs to be refilled from the drug several times per day, e.g. three to five times a day to meet an adult's typical TDD.

The present disclosure is based on the inside, that - in contrast to most adults - the filling volume of the pump cylinder of a dosing unit according to the general teaching of the EP 1970 677 A1 may me designed to be sufficient for the TDD of many diabetic infants without the device becoming bulky and without loosing the dosing precision advantage. In such a system, the drug container, which typically holds an initial volume of, e.g., 3 ml or 10 ml of insulin, is not part of the infusion device as such, but is provided separately and coupled to the infusion device only for refilling the pump cylinder, i.e. typically once a day.

In this way, the infusion device that needs to be carried at or very close to the infant's body can be considerably miniaturized. In addition, the insulin reservoir can generally be kept at a cool place, e.g. in a refrigerator, thereby expanding its use time and reducing the risk of using parity or fully degenerated and inactive insulin. As a further advantage, the amount of insulin that may - in a worst-case failure scenario - be accidentally infused into the infant's body, is largely reduced.

In a first aspect the present disclosure is directed to a dosing unit as claimed in claim 1 for an ambulatory infusion system, the ambulatory infusion system being designed for the infusion of a liquid drug into a patient's body over an extended time period. The dosing unit includes:

   a) an inlet port,
   b) an outlet port, the outlet port being configured to couple to the patient's body,
   and a pump kernel. The pump kernel includes
   c) a kernel body, the kernel body forming a limiting surface of a stepwise or continuously variable dosing volume,
   d) a control valve, the control valve being configured to couple the variable dosing volume alternatively, in a filling state, to the inlet port for filling or refilling the dosing unit by increasing the dosing volume, or, in a infusing state, to the outlet port for dosing liquid drug out off the dosing unit by deceasing the dosing volume.

The inlet port is configured for being repeatedly manually attached to and detached from an external drug container.

Thereby, a dosing unit in accordance with the present disclosure allows repeatedly carrying out a sequence of attaching the external drug container to the inlet port, filling the dosing unit with the control valve being in the filling state, detaching the drug container from the inlet port and infusing drug from the dosing unit with the control valve being in the infusing state. The variable dosing volume of the dosing unit, in combination with the control valve, forms a volumetric pump with the dosing volume serving as pump chamber.

Generally, changes of the infusion cannula - while necessary for medical reasons, especially in order to avoid or at least limit the tissue irritation caused by the cannula and to prevent complications such as severe inflammation - should be limited to the required minimum for a number of reasons: Cannulas and the corresponding tubing are cost critical disposables that are intended for a single application and contribute significantly to the overall therapy costs. In addition, the replacement process - while being doable without extensive medical training by a user at home on a regular basis, is time consuming and cumbersome. Finally, placing a new cannula tends to be painful in many cases and should therefore be reduced to the required minimum, especially for infants. Therefore, allowing the outlet port of the dosing unit to be coupled to the patient when refilling the dosing unit is a significant advantage of devices according to the present disclosure. During filling or refilling of the dosing unit, the outlet port needs to be safely blocked or decoupled to prevent any unintended drug administration. In the following, both initial filling and refilling of the doing unit is referred to as "filling" where no specific distinction is made.

In the following, reference is mainly made to the infusion of insulin to a diabetic patient by CSII where the teaching of the present disclosure may be used particularly favorably. This does, however, not imply a restriction to this specific application. The teaching of the present disclosure applies, mutatis mutandis, to the infusion of other drugs. For example, instead of insulin, growth hormones, pain killers, chemotherapy drugs and the like may be infused in an analogue way.

The dosing unit is typically realized as disposable product that is continuously used for a number of days and discharged afterwards. It may be fabricated by standard or multicomponent injection molding and/or other established large-scale manufacturing techniques. The single components of the dosing unit are typically made from plastics but may, fully or partly be made from further materials such as ceramics or metal.

The dosing volume may be varied between a maximum filling volume and a minimum filling volume that is favorably zero or close to zero, thus allowing expelling substantially the total liquid volume.

Within the context of the present disclosure, the phrase "continuously variable" also includes, besides a truly continuous variation, a variation in increments that are sufficiently small to be negligible from a medical point of view. A variation only between the minimum filling volume and the maximum filling volume without intermediate steps is not considered as "stepwise or continuously". Here and in the following, the phrase "filling volume of the dosing unit" generally refers to the volume that is enclosed in the variable dosing reservoir. The maximum filling volume of the dosing unit accordingly corresponds to a maximum value of the variable volume.

With respect to the pump kernel, the design may follow the general teaching of the EP 1970 677 A1, which further includes a number of design variants and a discussion of favourable operational conditions and safety characteristics of such a dosing unit. Based on the EP 1970 677 A1, further designs of pump kernels that may be applied in the context of the present disclosure are disclosed in the EP 2163273 A1 and the EP 2361646 A1 , respectively. However, various aspects may also be realized generally differently, as will be discussed below. includes at least one of a self-sealing piercable septum, a self-closing fluidic coupler, and a check-valve. Such design can ensure that the inlet port is hermetically sealed, thus preventing contamination of the dosing volume, as long as external liquid drug container is attached.Alternatively, another type of connector, such as a Luer connector, may be provided. A slider, a cap, or the like may be provided to protect the inlet port and prevent contamination. Such element may either be designed to close automatically upon detachment of the external drug container, or to be closed manually.A valve, such as a check valve, may further be provided as part of the inlet port or the fluidic channel between inlet port and vale assembly for safety reasons. Since the dosing unit is intended to be filled by drawing drug into the variable volume via negative pressure, the valve may be designed to open only if a corresponding pressure gradient over the valve from the inlet port side (higher pressure) to the control valve side (lower pressure) is present.

In alternative embodiments, the dosing unit is filled by forcing drug into the dosing volume via positive pressure (over pressure).

In an embodiment, the outlet port is realized as releasable fluidic coupler, such as a Luer connector that is coupled to an outlet side of the control valve via a fluidic channel. In such an embodiment, an infusion cannula is coupled to the outlet port via infusion tubing with a corresponding counter connector (e.g., a female Luer connector if the outlet port of the dosing unit is realized as male Luer connector). Alternatively, infusion tubing may be formed integral with the outlet port without a special coupler. In both cases, an infusion cannula may be formed integral with the tubing or coupled via a releasable cannula coupler. In a further variant, coupling of the dosing unit to the patient is realized without tubing. In this case, the outlet port includes or couples to a cannula directly or via a releasable coupler, e.g. via a pierceable septum. In such an embodiment, the ambulatory infusion system which is - as a whole - discussed below in more detail, is favorably designed to be adhesively attached to the patient's body, either directly or via an intermediate element, such as a mounting plate or cradle as will be discussed in more detail further below.

In some embodiments, the dosing unit is designed to be continuously couple, via the outlet port to the patient's body while filling the dosing unit. For this type of embodiment, the dosing unit stays coupled to the patient's body for its whole time of application, e.g. a week, and is removed only for replacement.

Alternatively or additionally, the dosing unit may be designed for fluidic decoupling the outlet port from the patient's body prior to filling the dosing unit and re-establishing the fluidic coupling to the patient's body after the filling. In particular for the patient being a baby or infant, it may be advantageous to remove the dosing unit for the (re-)filling process. For such embodiments, the outlet port may include at least one of a self-sealing piercable septum, a self-closing fluidic coupler, a check-valve or another assembly for ensuring hermetic sealing as generally described before in context of the inlet port. In some embodiments, the dosing unit has a maximum filling volume corresponding to the TDD of insulin of a diabetic infant, in particular a maximum filling volume in a range of 8 to 15 IU (International Units) of liquid insulin. For the typical insulin concentration U100, the resulting absolute volume is in a corresponding range of 0.08 ml to 0.15 ml. However, other liquid insulin formulations of lower or higher concentration, such as U40 or U200, may be used as well.

In some embodiments, the maximum filling volume matches a TDD of the patient. For the drug being insulin and the patient being a diabetic infant, the TDD corresponds to the above-discussed range of 8 to 15 IU. In a typical example, the maximum filling volume may be 10 IU, corresponding to 0.1 ml of liquid U100 insulin formulation. Being based on the total daily drug does means that the maximum filling volume corresponds to the total daily drug dose of an infant, favorably including a safety margin.

The total filling volume should be designed to match the largest Total Daily Dose (TDD) of an infant for which the dosing unit is intended. For an infant having a smaller TDD, the maximum filling volume is favorably not fully exploited. That is, the doing unit is favorably always filled to the TDD of a specific patient, again including some safety margin.

For this type of embodiment, the dosing unit needs to be refilled once a day, e.g. every morning or evening. Since the filling of the dosing unit only takes a comparatively small amount of time, typically in a range of some minutes, the dosing unit is in the filling state once a day for typically some minutes and is in the infusing state the rest of the day.

In some embodiments, the control valve is designed to switch between the filling state and the infusing state without displacement of a biologically significant amount of liquid drug or even without any drug displacement. Since drug infusion is controlled via stepwise or continuous reduction of the dosing volume in the infusing state, any displacement of drug, and in particular any displacement of drug into the outlet port and therefore into the patient's body during valve switching results in an unintended drug administration. In particular for patients with low TDD, such as infants, the involved amount of drug may be biologically significant if no special care is taken.

The control valve is favorably designed to sealing close the inlet port when the outlet port is coupled to the dosing volume and to sealing close the outlet port is coupled to the dosing volume. In particular the latter is required to prevent any unintended drug administration during filling of the dosing unit.

The control valve may especially be designed as rotary valve or sliding valve. Rotary valves and sliding valves are based on the principle of establishing and interrupting fluidic connections by aligning corresponding apertures in two members to overlap or misaligning them not to overlap via a relative rotary or linear movement of the two members. As compared to alternative valve designs that are based on opening or closing a flow channel by displacing a valve member inside the flow channel, rotary valves or sliding valves are particular favorable with respect to liquid displacement. A basic general design of a rotary vale that may be used in the context of the present disclosure is the a valve arrangement as shown in Figure 1 and disclosed in the EP 1970 677 A1 as discussed above. Other types of micro valves, such as miniaturized tube-squeezing valves, may generally be used as well.

In some embodiments, the control valve is designed to pass, when switching between the filling state and the infusing state, an intermediate vented state where the dosing volume is fluidic coupled to the environment. While generally unintended, some pressure offset may built up during application inside the variable volume of the dosing unit. By passing an intermediate vented state each time the control valve is switched between the filling state and the infusing state, such pressure offset is regularly equalized. In the vented state, the variable volume may especially be connected to the atmosphere or, for example, to an inner volume of an infusion device. The coupling may be direct or via a barrier membrane. An exemplary embodiment of a suited rotary valve is disclosed in the EP 2 457 602 A1, where conduits, grooves, and/or recesses are provided for fluidic coupling to the environment.

In some embodiments, the control valve is designed such that no fluidic path exists within the control valve between its inlet side and its outlet side that does not cross a fluidic connection to the environment. Thereby, a drain is establishing to the environment. This drain ensures that no liquid may flow directly from the drug container to the outlet port and accordingly to the patient during filling or refilling in case of a leakage or other defect of the control valve, even if an unintended positive pressure is present at the inlet side. A corresponding exemplary design that may be used in the context of the present disclosure is also disclosed in the EP 2 457 602 A1.

In some embodiments, the control valve includes the kernel body as a movable valve member and a further stationary valve member, the control valve being switchable between the filling state and the infusing state by moving the kernel body with respect to the stationary valve member.

In such an embodiment, the stationary member may include a fluidic inlet channel and a separate fluidic outlet channel in form of bores, apertures, etc, that are coupled to or integral with the inlet port and the outlet port, respectively. The engagement between kernel body and the stationary member should be fluidic tight or sealing, which may be achieved by hard and clogged mating surfaces of both kernel body and stationary member (hard-hard-sealing), or by sealing soft components, such as rubber or an elastomer. In some embodiments, soft sealing components may be formed integral with the kernel body and/or the stationary member, e.g. via two-component injection molding.

The kernel body may include a single kernel body aperture in a wall of the kernel body in fluidic communication with the dosing volume. For such an embodiment, the valve assembly is in the filling state if the kernel body aperture is aligned with the fluidic inlet channel and is in the infusing state if the kernel body aperture is aligned with the outlet port. Corresponding exemplary designs are disclosed in the EP 1970 677 A1, EP 2163273 A1, EP 2361646 A1, and the EP 2 457 602 A1.

Alternatively, the kernel body may include a kernel body inlet aperture and a separate kernel body outlet aperture. In such an embodiment, the kernel body outlet aperture is closed when the kernel body inlet aperture is aligned with the fluidic inlet channel while the kernel body inlet aperture is closed when the kernel body outlet aperture is aligned with the fluidic outlet channel. For such an arrangement, an intermediate vented state may be realized by providing a further channel in the stationary member that is fluidic coupled to the environment and is temporary aligned with a or the kernel body aperture when switching between the filling state and the infusing state. Corresponding exemplary designs are disclosed in the EP 1970 677 A1.

In some embodiments, the pump kernel is a piston pump, with the kernel body being a pump cylinder and the pump kernel further including a piston, the piston being displaceable, in particular linearly displaceable, arranged in a cavity of the pump cylinder, such that the surfaces of the cavity and the piston, in combination, define the dosing volume.

A piston pump has the particular advantage that its filling volume is in linear relationship with position and displacement of the plunger inside the cavity, thus providing good control of the pumping volume via the plunger displacement. The resolution of such a pump is only limited by the displacement resolution of the plunger. The piston of such a pump kernel may have a threaded section, e.g. an outer thread, and the pump cylinder may have a corresponding counter-threaded section, e.g. an inner thread inside the cavity, such that the piston is displaced in a screw-like motion inside the cavity upon a driving torque being applied. Corresponding exemplary designs are disclosed in the EP 1970 677 A1, EP 2163273 A1, EP 2361646 A1, and the EP 2 457 602 A1.

Suited dimension may be derived from the maximum filling volume. With the cylinder diameter being in a typical range of about 3 mm to 8 mm, the corresponding plunger displacement is in the cm-range.

In alternative embodiments, however, different type of pump chambers may be used. The kernel body may, for example, be realized as a fully or flexible pouch or bag that is mechanically compressed for reducing its volume and expanded for increasing its volume. In such an embodiment, no separate piston is present.

In some embodiments, the pump kernel includes a drive coupler for releasable coupling to a drive unit with a single drive, the dosing unit being designed to control force and/or torque transmission such that - during application - operation of the single drive alternatively effects both varying the dosing volume and switching the control valve between the filling state and the infusing state.

For the pump kernel being designed as piston pump, force and/or torque transmission may especially be controlled in dependence of a motion direction of the drive and/or the displacement position of the piston inside the cavity of the pump cylinder. Suited exemplary designs of releasable drive couplers are disclosed in the EP 2163273 A1, EP2361646 A1

This type of embodiments accordingly allows using a single drive only in the infusion system for both functions of valve switching and varying the dosing volume. Such a single-drive-design is considered to be particular favorable with respect to size, since only a single actuator is present. In embodiments of the pump kernel as discussed above, the drive may be a rotary drive and couple to the piston, in particular to an elongated piston shaft, only. Since the piston, when being displaced inside the cavity moves in a screw-like way, the coupling shall transmit a drive torque only without applying an axial force. That is, the drive coupler may couple the drive and the piston shaft in axial sliding engagement. Corresponding exemplary designs are disclosed in the EP 2163273 A1 and the EP 2361646 A1.

In alternative embodiments, however, the pump kernel includes a drive coupler for releasable coupling to a drive unit with a dosing drive for varying the dosing volume and a valve drive for switching the control valve between the filling state and the infusing state, the valve drive being separate from the dosing drive.

Providing separate drives for varying the variable volume and for switching the state of the control valve is considered may be favorable under aspects such as electromechanical system complexity and reliability as well as energy consumption since each drive may be optimized for its dedicated function.

In a variant, the dosing unit is designed to couple to a separate drive for filling and emptying the dosing unit only, i.e., for varying the variable volume, while the control valve is designed for manual switching by a user. In contrast to an infusion system for adults, where the dosing unit needs to be refilled several times a day, manual valve switching is feasible in the therapy of infants, where valve switching is required only once a day in the context of filling the dosing unit which requires presence of a responsible user and a number of manual actions anyway.

In some embodiments, the dosing unit includes a user-operable cradle-coupler, the cradle coupler being designed to engage a dosing unit coupler of a separate skin-mountable cradle, thus enabling attachment of the dosing unit to the patient's body via the cradle. Typically, a suited cradle is made by a substantially sheet-like plastic structure and has, on its proximal side that Is intended to contact the skin, an adhesive coating, film, or layer for temporary skin attachment. The dosing unit coupler is typically arranged on the adjacent distal side of the cradle. The cradle-coupler and the dosing unit coupler may be designed for releasable or non-releasable coupling. In this context, "releasable" means that the dosing and the cradle and the dosing unit may be physically separated and again attached to each other be re-establishing engagement, i.e. that a sequence of repeated engagement and disengagement may be carried out by the device user and without damaging either of the dosing unit or the cradle. The cradle unit may further include a transcutaneous infusion cannula or an infusion cannula coupler for coupling with an infusion cannula. A cradle that may be modified for use in combination with a dosing unit according to the present disclosure is described, e.g. in the WO 2009/06635 A2.

According to a further aspect, the present disclosure is directed towards an ambulatory infusion systems. An ambulatory infusion systems in accordance with the present disclosure may include

   a) a dosing unit as described above,
   b) a base unit, the base unit including

   • a drive unit, the drive unit being designed to releasable couple to the dosing unit,
   • an electronic control unit, the control unit being coupled to the drive unit for controlling operation of the infusion system alternatively in a filling mode for filling, by increasing the dosing volume, the dosing unit with liquid drug from the drug container, and in an infusing mode for infusing - by decreasing the dosing volume - liquid drug into the patient's body.

In some embodiments, the ambulatory infusion system includes a number of more than one dosing unit that may be sequentially used in combination with the same base unit. In a typical embodiment, the dosing unit has a use time of some days while the useful lifetime of the base unit may, e.g., be about six months or even several years. Dosing units may generally be provided together with and/or separate from the base unit.

The dosing unit and the base unit may each have a housing which are coupled for the application via elements such as snappers, hooks, latches, and the like. Alternatively, the base unit may have a housing with a dosing unit compartment that receives the dosing unit for the time of its application. In such an embodiment, the dosing unit compartment may be accessible via a hinged door, a slider, a removable cover, or the like.

The control unit may generally be designed according to design rules known from state-of-the-art infusion devices, including components such as microcontrollers, memory units, power control circuitry, clock units, safety circuitry, and the like. The control unit may further include or be coupled to a user interface, the user interface including output components such as a display, a touch screen or the like, and input components such as push buttons, jog wheels, multi-direction-switches, and the like.

In embodiments that are used as insulin pump, the control unit is designed to control the drive unit, in the infusing mode, to infuse insulin both according to a time variable basal infusion schedule and to infuse additional insulin boli on demand.

The drive unit typically includes one or multiple rotary drives, such as standard DC motors, electronically commutated (brushless) ECDC motors, stepper motors, or the like. Alternatively or additionally, other types of actuators, such as electromagnets or shape memory alloy SMA actuators may be used.

In embodiments where the control valve is not switched manually but via the drive unit, the control unit is designed to operate the drive unit to switch the valve arrangement from the infusing state into the filling state prior to increasing the dosing volume in the filling mode and from the filling state to the infusing state prior to decreasing the dosing volume in the dosing mode.

In some embodiments, the functionality of the control unit is distributed between a number of separate and distinct devices. In particular, a separate remote control device may be present that partly or fully includes the user interface and is coupled to a patient-based device and especially includes the drive unit and the dosing unit by wireless connection, e.g. via Bluetooth®. Such a remote control device may also include further functionality such as a blood glucose meter, a food database, an insulin bolus recommendation system, statistics and therapy evaluation functionality, a coupling interface to further devices such as a PC and/or a cell phone net, a coupling interface to a continuous glucose monitoring system, and the like.

In the context of therapy of infants, the remote control device may generally be carried by a responsible adult. Therefore alarms that are indicative of a hazardous situation, such as a cannula occlusion, a leakage or a device error, may generally be indicated on the remote control device in addition or alternatively to the patient-based device.

In some embodiments, the control unit is further designed to activate an alarming device when, during operation in the infusing mode, the filling state of the dosing kernel approaches emptiness, i.e. the remaining filling volume of the dosing kernel approaches zero.

This may be detected by counting, after filling or refilling the dosing unit to a known volume, an accumulated amount of infused drug. For such an embodiment, the alarming device may be activated upon the accumulated amount of drug passing or exceeding a threshold level. For an exemplary maximum filling volume of the dosing unit being 10 IU, the threshold level may, e.g., be 8.5 IU, 9 IU, or 9.5 IU. Alternatively, the system may include a volume sensing unit that determines the remaining filling volume. In case of the dosing unit including a piston pump, the sensor may, e.g., be a position sensor for the displacement position of the plunger inside the cavity of the kernel body. In any case, the alarm should be provided sufficiently early to safely allow refilling of the dosing unit (or replacement of the dosing unit) before it actually is empty. Providing an alarm about 0.5 hour to 2 hours before actual emptiness of the dosing unit is considered appropriate.

Alternatively to a single alarm, an number of alarms or warnings may be provided at different remaining filling levels of the dosing unit. In some embodiments, the current filling volume of the dosing unit may be indicated at any time, e.g. on a display.

The alarming device of such an embodiment may be an audio alarming device, such as a loudspeaker or buzzer, and/or a tactile alarming device, in particular a pager vibrator, that may be integral with the control unit. In particular in the context of treatment of infants, the alarming device is favourably part of a remote control device that is carried by the responsible adult, such as a parent. Alternatively or additionally, a cell phone may serve as alarming device and the control unit may be designed to transmit the warning to the cell phone via an available cell phone net, e.g. via SMS. Alternatively or additionally, a portable alarming device as well as the control unit may be integrated in a local home network and the alarm may be transmitted from the control unit to the alarming device via such a network.

In embodiments where the type and in particular the initial filling volume of the drug container is known, the control unit may be designed to monitor the total drug amount that is drawn from a specific drug container in an number of dosing kernel fillings and may be designed to provide an alarm if the drug container approaches emptiness, e.g. at a remaining filling level that is sufficient for filling the dosing kernel one or two further times.

In some embodiments, the control unit is further designed, before switching the ambulatory infusion system to the infusing mode, to request a user confirmation input. Such a user confirmation at the end of a dosing unit filling or (re)-filling procedure may be provided for safety reasons in order to make a user, in particular a responsible adult, aware of the starting infusion. The user may further be requested to manually verify and confirm that the dosing unit is actually filled with drug.

In some embodiments, the base unit includes a coupling detector, the coupling detector being coupled to the control unit, to provide a signal indicating whether or not a drug container is coupled to the inlet port. In such an embodiment, the control unit is further configured to control the drive unit to increase the variable volume, thus drawing drug from the drug container into to dosing unit, only if a drug container is coupled to the inlet port.

Increasing the variable volume without a drug container being attached would result in filling the dosing unit with air rather than with drug.

The coupling sensor may be realised as electrical switching contact, such as a micro switch, that is actuated upon a drug container being coupled to the inlet port. In a similar way, a reed switch may be used. In a more advanced embodiment, the drug container or a container storage box comprising the drug container includes a wireless transmitter, such as an RFID tag, and the base unit includes a corresponding reader unit that is coupled to or integral with the control unit to receive a signal transmitted by the wireless transmitter. Such an embodiment may be designed to provide a higher level of safety as compared to a simpler switching device and may further identify a particular drug container or storage box.

In addition, an RFID tag may store further useful data, such as a lot number and/or expiry date of the drug container, and/or the amount of drug still present in the drug container. Such information may be transmitted to the control unit of the infusion system where the information is evaluated and messages, warnings, alarms etc may be generated where appropriate.

In some embodiments, the ambulatory infusion system further includes a container storage box, the container storage box being a self-contained unit, the container storage box including:

    a) a drug container or a compartment for replaceable receiving a drug container,

    b) a temperature control unit, the temperature control unit being designed to vary a temperature of liquid drug that is stored inside the drug container in accordance with a schedule by actuating an electrically driven heating and/or cooling element in accordance with a time-varying schedule.

The temperature control unit is realized as electronic unit, typically including control circuitry, a power supply such as a rechargeable or non rechargeable battery and/or a power line adapter and a temperature sensor for the drug container temperature. Besides its application in controlling the drug container temperature, the temperature sensor may be used to provide an alarm if the drug container temperature exceeds or falls below specified threshold limits.

As electrically driven heating element, one or more electro-resistive elements, such as ohmic resistance elements may be provided. Alternatively or additionally Pelletier-elements may be present that may be controlled to alternatively operate both as heating and/or cooling element. Additionally or alternatively, devices for convection improving, in particular a fan, may be present.

The drug container may, e.g., be a standard 10 ml insulin vial with a piercable septum. Alternatively, the drug container may be a so called pen cartridge of typically 3 ml volume, the pen cartridge having a generally cylindrical body that is closed at one front surface by a piercable septum and further includes a sealing displaceable plug inside the cartridge body for displacing, by pushing the plunger towards the septum, drug into a cannula piercing the septum. Other forms of drug containers, such as fully or partly flexible bags or pouches, may be used as well.

Favorably, the drug container or the container compartment is designed such that a fluidic outlet of the drug container can be coupled with the inlet port without removing the drug container from the storage box. In case the drug container has a septum, such as vial or a pen-cartridge, a casing of the storage box may have an aperture that may be closable by a cap, a slider, etc, through which a container portion with the septum projects or is accessible, thus allowing access to the septum. Mating coupling elements, such as guides, a bayonet etc. may be present to allow safe coupling with corresponding counter-parts of the dosing unit or the base unit of the infusion system.

In some embodiments, the storage unit includes or is designed to receive a drug container of fixed inner volume, such as a standard insulin vial. In those embodiments, the storage box may include or be designed to receive a pressure equalization device to prevent the built-up of negative pressure inside the drug container when drug is draw from the container. A pressure equalization device may, e.g., be formed by a venting cannula that pierces a septum of the drug container and fluidic couples the storage volume of the drug container to the environment, for example via a fluid-tight and air-permeable membrane. Such a pressure equalization device may be provided as separate disposable element that is generally replaced with the drug reservoir.

For drug containers that allows decreasing their storage volume when drug is drawn, such as a bag or pouch, no negative pressure will generally build up and a pressure equalization device may not be required, but may be optionally be provided as well. In particular in embodiments where the drug container is a pen-type cartridge of potentially high plunger friction, drawing liquid may be difficult or even impossible because the plunger needs to be displaced by a negative (sucking) pressure that builds up between drug and plunger upon drug being drawn. In those cases, a biasing device may be present in the storage container to exert a biasing pushing force onto the plunger. The biasing device may, for example, include a compression spring that is arranged inline and behind the drug container, thus exerting a biasing force.

An air retention device or an air removal device as generally known in the art may be present as part of the drug container or the storage, box, i.e. in form of a disposable element, in order to prevent an entry of air that is originally present in the fluidic system and/or is dissolved in the liquid drug, into the dosing unit. As discussed above, any unintended administration of air is particularly critical in the therapy of infants.

In some embodiments, the storage box or the drug container inside the storage box are designed for direct coupling with the dosing unit. This is the case, for example, in embodiments where one of the drug container and the inlet port of the dosing unit includes a piercable septum and the other of the drug container and the inlet port of the dosing unit includes a corresponding piercing cannula, or if further releasable couplers, such as Luer connectors, are provided. Alternatively, the storage box and the dosing unit may be designed to couple via an adapter that is typically disposable and only used for a single filling of the dosing unit. An appropriate exemplary adapter includes an adapter body that is typically made form plastics, and a fluidic channel. In case of both the dosing unit and the drug container having a piercable septum as fluidic interface, for example, the adapter may include a double-pointed hollow cannula that is hold by the adapter body for piercing both septa. The adapter, in particular the adapter body, may further include guiding and/or mating coupling structures such as bayonet elements, elastic snapping elements, etc, to provide and ensure safe coupling. In those embodiments, the dosing unit and/or the base unit as well as the drug container and/or the storage box include corresponding counterpart coupling structures. The adapter may further include an air retention or air removal device and/or a pressure equalization device as discussed above.

In some embodiments including a storage box, the temperature control unit is configured to control the temperature of the drug container such that the temperature of liquid drug that is stored inside the drug container increases at points in time that are correlated to points in time for filling or the dosing unit.

This type of embodiment accordingly allows storing the drug generally at a suited recommended storing temperature that is typically below room temperature, e.g. in a range of 8°C to 12°C for a typical liquid insulin formulation. As discussed above, this is particularly desirable in the therapy of infants where a single drug container, such as a 10 ml insulin vial, may store sufficient insulin for several weeks of therapy.

While this may generally achieved by simply storing the drug container at a cool place of appropriate temperature, e.g. in a cellar or - most typical - in a refrigerator, a storage box in accordance with the present disclosure allows increasing the drug temperature some time prior to filling or refilling the dosing unit, which has a number of advantages. The temperature may especially be increased to substantially equalize with the ambient room temperature or with body temperature.

This type of embodiment has particular advantages. Firstly, refilling the dosing unit with the drug being at room temperature avoids or reduces the well-known problem of air being dissolved in the liquid drug and outgassing inside the dosing unit after refilling. While the injection or infusion of air is generally undesirable, the dosing error resulting from injecting or infusion air instead of liquid drug is particular critical for infants because an identical absolute amount of air and, accordingly, an identical absolute under-dosing of drug corresponds to a considerably higher relative under-dosing for infants because of their lower TDD. Secondly, the injection or infusion of cold liquid drug - which would normally occur at least for some time after refilling of the dosing unit - is known to be particularly painful.

In some embodiments, the temperature control unit includes at least one of

- a clock relay, the clock relay controlling the heating and/or cooling element, or
- a receiver, the receiver being configured to operatively couple to the control unit and to receive from the control unit a temperature control signal for varying the temperature of liquid drug inside the drug container.

Providing a clock relay that controls the heating and/or cooling element is based on the assumption that - in particular in the therapy of infants - the TDD is substantially constant and that refilling of the dosing unit may accordingly be virtually always done at approximately the same time of day for all days. For such an embodiment, the clock relay may accordingly be programmed to start increasing the drug temperature some time prior to the scheduled time for refilling the dosing unit, such that the drug has the desired temperature when refilling of the dosing kernel is actually carried out.

In somewhat more complex alternative embodiments, the drug temperature is varied based on a temperature control signal received from the control unit of the infusion system, thereby providing additional flexibility with respect to day-to-day variation of the insulin demand. The temperature control signal may directly correspond to the desired temperature or temperature change of the drug or may be a trigger signal for starting a temperature variation, in particular a temperature increase. Wireless transmission of the temperature control signal may be carried out using typical RF technologies, such as Bluetooth®. Alternatively, a local home network may be used for transmitting the temperature control signal.

In a further variant, a temperature control signal is transmitted from the control unit of the infusion system to the temperature control unit of the storage box when the storage box is coupled to the dosing unit for refilling. In such an embodiment, the temperature control signal may provide an expected time for the next following refilling of the dosing unit. Once the temperature control signal is received by the temperature control unit, further communication is not required and the temperature control module may operate in accordance with the previously received information. In such an embodiment, wireless near-filed communication, based, e.g. on direct capacitive or inductive coupling, may alternatively be used for transmitting the temperature control signal. In a further variant, the temperature control signal may be transmitted via galvanic coupling.

In some embodiments, the storage box is designed to be generally stored at room temperature. Since room temperature is typically above the drug storing temperature, it is generally sufficient to provide a cooling element such as a Pelletier element, optionally in combination with a miniaturized fan. For this type of embodiment, active cooling of the drug container is necessary most time of the day, which is generally feasible if the storage box is supplied with electric energy via a power line adapter. If the storage box is battery operated, energy consumption may be critical for such an embodiment. Even though not absolutely necessary for this type of embodiment, a heating element may be provided in addition to the cooling element in order to reduce the time required for the temperature increase and/or to more precisely control the temperature increase, independent of the current room temperature.

In some embodiments, the ambulatory infusion further includes a skin-mountable cradle, the cradle being separate from the dosing unit, wherein at least one of the dosing unit and the base unit includes a user-operable cradle coupler and the cradle includes a user-operable device coupler, the cradle coupler and the device coupler being designed for mutual engagement, thus enabling attachment of the dosing unit to the patient's body via the cradle.

The cradle unit may especially a cradle unit that is designed for temporary adhesive attachment to the patient's skin as generally described above and may, e.g. be designed in accordance with the disclosure of WO 2009/06635 A2 In some embodiments including a cradle, the device coupler and the cradle coupler are designed for a repeated sequence of engagement and disengagement.

In some embodiments, the container storage box is designed to be generally stored, during application, in a refrigerator. In the context of the present disclosure, the term "refrigerator" is used in the sense of the typical household home appliance, but may also be another refrigerator-like device that may be generally available in a household, such as a camping cooling box.

This type of embodiment allow the generally required cooling to be carried out by the refrigerator, i.e. by a device external to the storage box. For this type of embodiment, only a heating element may be provided in order to increase the drug temperature prior to refilling the dosing unit. The temperature control unit may be designed to increase the drug temperature while the storage box is included in the refrigerator.

This type of embodiment is particularly favorable since it allows removal the storage box from the refrigerator only immediately prior to usage. With respect to energy consumption, however, this type of embodiment appears to be less favorable since the heating element needs to overcome the continued cooling by the refrigerator. Therefore, in a variant, the storage box is designed to be generally stored in a refrigerator but to be removed from the refrigerator prior to the heating element increasing the drug temperature. In such an embodiment, the storage box and/or the control unit of the infusion system may be designed to provide a dedicated alert for removing the storage box from the refrigerator. Such an alert may be generated directly by the ambulatory infusion system, e.g. via an alarming unit as discussed above, and/or may be generated by a remote device, such as a remote controller or a cell phone. In this case, a corresponding alert signal may be generated by the control unit of the infusion system or the temperature control unit and wirelessly transmitted to the remote device as discussed above.

In all of the above-described embodiments of a storing box, a further temperature sensor may be present for sensing the surrounding temperature. This allows adopting the temperature increase by a heating element to varying room temperatures. In embodiments where the storage box is generally stored in a refrigerator, such a temperature sensor may also be used by the temperature control unit to adapt for varying refrigerator inside temperatures as well as different storing places for the storing box inside the refrigerator, which typically have different temperature.

A storage box as described above is considered to be of use also beyond the context of an infusion system in accordance with the present disclosure. Rights are explicitly reserved for seeking protection for a storage box according to any of the embodiments as described above or below in the context of specific examples. Such a storage box may be used in combination with any type of ambulatory infusion device, such as a portable insulin pump like the Accu-Chek® Spirit Combo pump, available from Roche Diagnostics, or similar devices.

In such a storage box, the drug reservoir and/or the storage box might be designed for coupling to any external receiving device that shall receive drug from the drug reservoir, such as a cartridge, bag, pouch, or the like for use in an infusion device, a drug reservoir of a patch-like infusion device, or a single-use syringe.

In a further application, a storage box in accordance with the present disclosure may be used without an infusion system and in combination, e.g., with ordinary standard syringes, as still common in many countries, e.g., for insulin injections. The storage box is generally useful where a liquid drug should generally stored at a different - typically lower - temperature as compared to the temperature for injection or infusion.

In the context of a storage box, a method for storing a drug container, the drug container being filled with a liquid drug, is further disclosed. Such a method may include the steps of

> a) providing a storage box, the storage box including the drug container as integral component or replaceable received in a drug container compartment, the storage box further including a temperature control unit, the temperature control unit including an electrically driven heating and/or cooling element, and

> b) operating the temperature control unit to vary a temperature of the liquid drug by actuating an electrically driven heating and/or cooling element in accordance with a time-varying schedule that is stored in or received by the temperature control unit.

In a method according to the present disclosure, the external receiving device may, for example, be a dosing unit according to the present disclosure as discussed above, and the drug reservoir port may accordingly be designed for repeatedly fluidic coupling the drug container to and decoupling the drug container from the inlet port of such a dosing unit.

Since a method according to the present disclosure may especially be carried out with a storage box according to the present disclosure, exemplary embodiments of a storing box as given above and further below disclose, at the same time, corresponding embodiments of storing a drug container, and vice versa.

**Exemplary embodiments**

In the following, exemplary embodiments of devices, systems in accordance with the present disclosure are explained in more detail with additional reference to the figures.

> Figure 1 (discussed above) schematically shows an exemplary pump kernel of a dosing unit in accordance with the present disclosure.

> Figure 2 shows an exemplary infusion system in a schematic functional view together with further associated elements.

> Figure 3 shows a further exemplary infusion system in a schematic functional view together with further associated elements.

> Figure 4 shows a container storage box in a schematic functional view together with further associated elements.

> Figure 5 shows an exemplary method for storing a drug container.

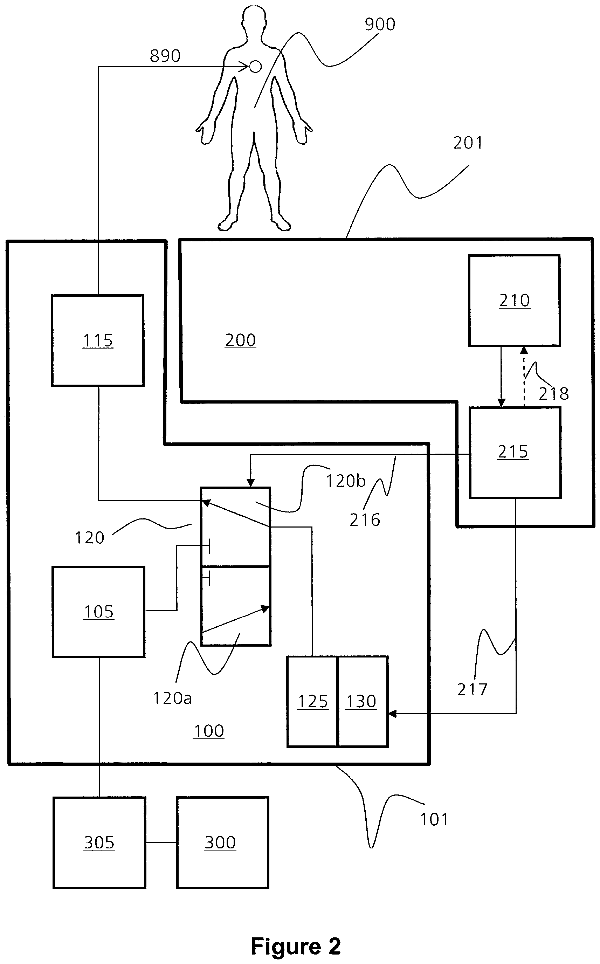

The exemplary infusion system as shown in Figure 2 generally includes dosing unit 100 and base unit 200. Dosing unit 100 includes inlet port 105, outlet port 115 and a pump kernel with control valve 120a, 1 2b, pump cylinder 125 and piston 130. Base unit 200 includes control unit 210 and drive unit 215.

In this example, dosing unit 100 and base unit 200 are each shown with separate casings, 101 and 201, respectively, which are designed for releasable coupling in a situation of use as discussed above. Alternatively, however, dosing unit casing 101 may also be removably received in a corresponding compartment of base unit casing 201.

Inlet port 105 is, in this example, realized by a piercable septum, thus allowing repeated and releasable coupling to drug container 300, which is, e.g., a standard 10 ml insulin vial. Drug container 300 couples to inlet port 105 via adapter 305. Adapter 305 is provided as separate single-use disposable and includes a double-pointed needle to pierce the septum of inlet port 105 and a septum of drug container 300. Optionally, adapter 305 further includes an additional venting cannula (not shown) that vents the inner volume of drug container 300 to atmosphere and/or an air removal device and/or an air retention device (not shown).

Outlet port 115 may be designed as Luer connector that couples to patient 900, e.g. for example a diabetic infant, via infusion tubing 890 and an infusion cannula (not separately shown) which may be designed according to the state of the art.

Alternatively, outlet port 115 may be designed in a self-sealing way as generally discussed above in the context of the general description and may and e.g., include a self-sealing septum. In such an embodiment, infusion tubing 890 may be formed integral with an infusion cannula. In such an embodiment, infusion tubing/infusion cannula 890 may comprise a body that is typically made from plastics and from which a part of the infusion cannula that is designed to be introduced projects. In such an embodiment, infusion tubing 890 forms together with the body and the infusion cannula, a generally rigid compact unit that is designed for attachment, as a whole, to the dosing unit casing 101. The infusion tubing/ infusion cannula 890 of such an embodiment may further include a pointed outlet port coupler that is designed to pierce the septum of outlet port 115, thus establishing a fluidic connection. The body may further include a mechanical coupler for establishing a locking engagement with dosing unit casing 101. A suited design that may be modified for use in the present context is disclosed, e. g., in the WO 2004/110526 A1.

During application, the ambulatory infusion system with dosing unit 100 and base unit 200 may be designed for attachment to the body of patient 900 via a suited belt, or - in case of a small infant - next to the infant on a mattress etc. Alternatively, the system may be designed for direct adhesive skin-attachment. In such an embodiment, infusion may be carried out via infusion tubing 890 having a length in the range, e.g. of 0.3 m to 0.8 m, or via tubing 890 and infusion cannula being formed as integral compact unit as discussed before. In a further alternative, the combination of dosing unit 100 and base unit 200 is designed for indirect adhesive skin-attachment via a body-mountable cradle, as also discussed before. In a further variant, the dosing unit 100 may be formed integral with a cradle and be designed for releasable locking coupling wit the base unit 200.

The pump kernel of exemplary dosing unit 100 is designed as follows: Piston 130 is linearly displaceable arranged in a corresponding cavity (not referenced) of pump cylinder 125 such that the walls of the cavity and piston 130, in combination, define the dosing volume of the pump kernel. The overall design of pump kernel and control valve 120a, 120b is - for exemplary purposes - assumed to follow the teaching of one or more of EP 1970 677 A1, EP 2163273 A1, EP 2361646 A1, EP 2 457 602 A1.

Control valve 120a, 120b includes a stationary member (not referenced) and pump cylinder 125 as movable member, with pump cylinder 125 being rotatable about its longitudinal axis with respect tot the stationary member, thus switching control valve 120a, 120b between filling state 120a and infusing state 120b as described above. In filling state 120a, inlet port 105 couples to the cavity in pump cylinder 125 with a passage to outlet port 115 being sealing blocked. In infusing state 120b, outlet port 115 to the cavity of pump cylinder 125 with a passage to inlet port 105 being sealing blocked. Figure 2 exemplarily shows the control valve in the infusing state.

Optionally, control valve 120a, 120b may have one or more intermediate states (not shown) that are passed when switching between filling state 120a and infusing state 120b. Those intermediate states may include a vented state where the cavity of pump cylinder 125 is in fluidic connection to the environment and blocked states where the cavity of pump cylinder 125 is blocked.

Drive unit 215 exemplarily is realized by a single electric motor and a reduction gear, e.g. a planetary gear unit, a helical gear unit, or a combination of both. Drive unit 215 couples to piston 130 via a rotational coupler for transmitting a driving torque to piston 130 for both displacing piston 130 and switching the state of control valve 120a, 120b, according, e.g., to the disclosure of EP 2163273 A1. Torque transmission from drive unit 215 to piston 130 for linear displacement is indicated by functional link 217, torque transmission from drive unit 215 to control valve 120 is indicated by functional link 216.

Alternatively, drive unit 215 may transmit a torque to piston 130 for the piston displacement via functional link 217 and, separately, to pump cylinder 125 via functional link 216 for the valve switching. In a further alternative, drive unit 215 includes separate drives, such as two electric motors, for effecting piston displacement and valve switching. In a variant, functional link 216 between drive unit 215 and control valve 120 is not present. In such an embodiment, valve switching is carried out manually as discussed above.

Control unit 210 generally includes all circuitry and components for controlling and supervising operation of the infusion system as discussed above. Control unit 210 is in particular designed to control the infusion system to carry out drug infusion according to a time-varying basal schedule and to further administer drug boli on demand. In addition, control unit 210 may control the infusion system for the refilling of the pump kernel. Control unit 210 may further implement functions that are typically present in state-of-the-art ambulatory infusion systems and in particular insulin pumps, such as providing alarms/reminders, transmitting data to external devices, such as a PC for record keeping and statistic evaluation, and the like. While not separately shown for clarity reasons, a power supply, such as one or more rechargeable or non-rechargeable batteries, is typically also included in base unit 200.

Optionally, drive unit 215 provides one or more feedback signals to control unit 210 for control and/or supervision purposes. Suited feedback signals are, e.g., motor current, rotational motor speed, and/or angular motor shaft position. Therefore, corresponding sensors or encoders, such as one or more of tachogenerators, hall-encoders, and absolute or incremental optical encoders may be present.

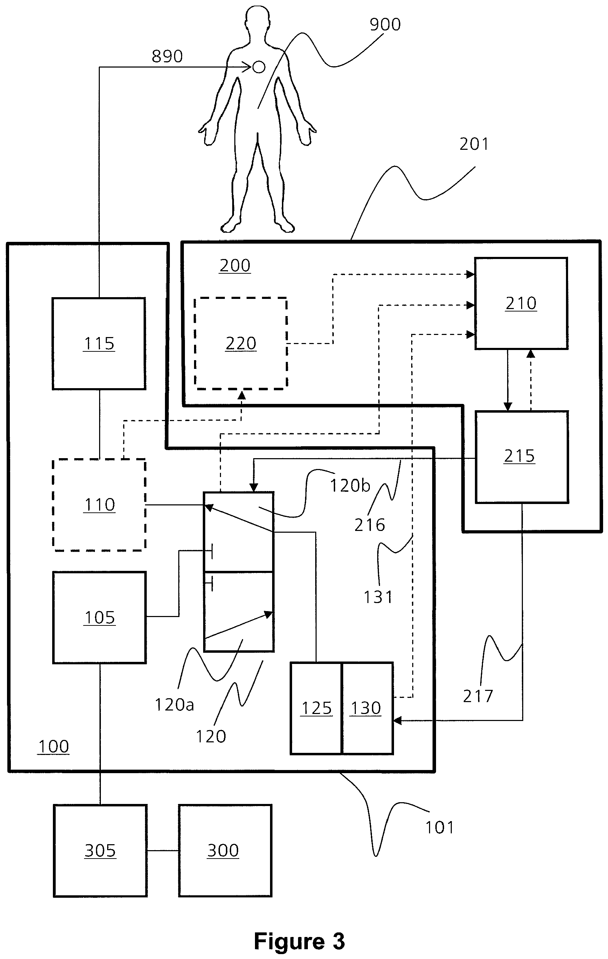

Figure 3 shows a further exemplary infusion system in accordance with the present disclosure in a schematic functional view. While the embodiment of Figure 3 shows a number of additional or modified features, the general design is the same as the embodiment shown in Figure 2 and as discussed above. The following discussion therefore focuses on the additional or modified features.

In the embodiment of Figure 3, a position feedback signal may be provided by piston 130 to control unit 210 for control and/or supervision purposes. Therefore, control unit 210 may include corresponding sensors, in particular a position sensor, e.g. a CCD sensor or Position Sensing Device PSD for determining the linear displacement position of piston 130. Alternatively, a shaft of piston 130 may include an encoder scale, e.g. in form of a colored pattern, that cooperates with corresponding optical transmitters and receivers of control unit 210, thus forming an incremental linear encoder.

In the embodiment of Figure 3, vale assembly 120a, 120b provides a feedback signal to control unit 210, the feedback signal being indicative of the valve state, for control and/or supervision purposes. Therefore, control valve 120a, 120b may include or couple to corresponding sensors, such as optical, electrical, capacitive, or magnetic switches.

Further in the embodiment of Figure 3, outlet port 115 couples with control valve 120b via optional fluidic sensor unit 110 and corresponding base sensor unit 220. While fluidic sensor unit 110 is shown as separate functional unit in Figure 3, it may, fully or partly, be realized integral with further components, in particular a stationary member of control valve 120a, 120b, e.g. as common injection molded component. Sensor unit 110, 220 may include a pressure sensor for determining a fluidic pressure at outlet port 115. A suited pressure sensor may, e.g., be based on determining and evaluating the deflection of a pressure transfer membrane under fluidic pressure. Membrane deflection may, e.g., be determined electro-capacitive or optical. Fluidic pressure measurement is particularly useful for rapidly detecting occlusions, that is, fluidic blockages of the fluidic channel, in particular in infusion tubing 890 and/or the infusion cannula (not shown). A further exemplary sensor that may be included in sensor unit 110, 220 is an air sensor for detecting air or gas bubbles that leave control valve. While liquid-contacting components of such sensors, e.g. a pressure transfer membrane and/or optical prisms, are favorably realized integral with the disposable dosing unit 100 as fluidic sensor unit 110, further components such as optical (IR) transmitters and receivers are favorably realized as base senor unit 220 that is part of part of base unit 200 or coupled to base unit 220. During application, fluidic sensor unit 110 and base sensor unit 220 releasably couple, using, e.g. mechanical and/or optical coupling. Signal processing and evaluation of the signals provided by base sensor unit 220 is carried out by control unit 210.

While the embodiment as shown in Figure 3 includes a number of additional option features, in particular feedback features, it is to be understood that they do not need to be necessarily present in combination but may or may not be present independent from each other.

Figure 4 shows a container storage box 400 in accordance with the present disclosure. In this specific example, container storage box 400 is part of an exemplary ambulatory infusion system as discussed above and shown in Figures 2 and 3. Alternatively, however, container storage box 400 may be part of a different infusion system or may be used as stand-alone device.

Container storage box 400 includes a storage box housing 401 with a container compartment 405. Container compartment 405 may be designed to receive any drug container 300 as discussed above. In the following, drug container 300 is considered to be an insulin vial of typically 10 ml filling volume. Container storage box 400 and drug container 300 allow coupling to a dosing unit as discussed above or generally a receiving device, such as a syringe.

Container storage box 400 further includes a temperature control unit with temperature controller 410 and electric heating and/or cooling element 415 that is driven and controlled by temperature controller 410. Container storage box 400 may be designed to be generally kept at a cool place, e.g. in a refrigerator. In this case, element 415 may be a resistor-based heating element. Alternatively, container storage box 400 is designed to be generally kept at room temperature. In this case, element 415 may be a Pelletier element for both heating and cooling. In any case, heating and/or cooling element 415 and container compartment 405 are designed to establish a good thermal coupling between drug container 300 and heating and element 41 5. Therefore, element 415 may be integrated into a wall of container compartment 405. Temperature feedback to temperature controller to temperature controller 410 is provided via optional temperature sensor 420 which is, like element 415, arranged in direct thermal contact with container 300. Optional ambient temperature sensor 422 may also be present.

In the exemplary embodiment of Figure 4, temperature controller 410 receives a temperature control signal via communication interface 407 for controlling heating and/or cooling element 415. Alternatively, the temperature control signal may be provided via wired galvanic connection. Optionally, temperature controller 410 provides feedback to control unit 210, e.g. via communication interface 407. In embodiments of the infusion system where control unit 210 is distributed between base unit and remote controller as will be discussed below, temperature controller 410 may be designed for communication with the base unit, the remote controller, or both. Alternatively or additionally, container storage box 400 may be designed for self-contained and independent operation. In this case, temperature controller 410 typically includes a clock relay.

Container storage box 400 may be deigned to be exclusively remotely controlled via control unit 210. In such an embodiment, container storage box 400 may not include any user interface such as switches, buttons, display etc. Such a design may especially be favourable if container storage box 400 is intended to be permanently kept in a refrigerator or the like. Alternatively or additionally, however, temperature controller 410 may have its own user interface.

In the exemplary embodiment of Figure 4, optional passive RFID tag 310 is attached to drug container 300. RFID tag 310 may be an integral part of drug container 300. A unique container identifier is stored by RFID tag 310 and may read out by a corresponding reader of control unit 210. Information stored by RFID tag 310 may optionally be communicated to control unit 210 via temperature controller 410 and communication interface 407, which in this case serve as relay. RFID tag 310 may also store further valuable data, such as an expiry date of the drug container, and/or the amount of drug still present in the drug container. The data may be evaluated by temperature controller 410, by control unit 210, or both.

An RFID reader in temperature controller 410 may also be used to automatically detect when a drug container 300 is removed and/or inserted into container compartment 405.

Figure 5 shows the major steps of an operational flow for a method of storing a drug container, e.g. drug container 300 in a storage box 400 as shown in Fig 4, storage box 400 being used in combination with an infusion system 200 as shown in Fig. 2, or Fig. 3, respectively. For the moment, it is assumed that container storage box 400 is normally stored in a refrigerator and that element 415 is a heating element. As can be seen, the operational flow includes a first main block with steps S1, S3 and a second main block with steps S5, S7.

Step S1 is a waiting step where container storage box 400 is stored, including container 300, at a location of suited low temperature, such as a cellar or refrigerator, and heating element 415 is switched off. In step S3, the reception of a temperature control signal by communication interface 407 - the temperature control signal being wirelessly transmitted from control unit 210 and being provided some time before refilling of pump cylinder 125 is required -is tested. In case of no corresponding signal being received, operational flow continues with executing waiting step S1.

If a corresponding temperature control signal is received, operational flow branches to warming step S5. In warming step S5, heating element 415 is activated to warm container 300 to room temperature and to maintain it at this temperature until refilling of pump cylinder 125 is carried out. A desired target temperature to which the liquid in container 300 shall be warmed up may be stored in temperature controller 410 or transmitted as part of the temperature control signal. A variety of generally known control algorithms may be applied in step S5. In a basic embodiment, a two position controller algorithm may be implemented in temperature controller 410, with feedback being provided by temperature sensor 420.

In the exemplary embodiment, storage box itself 400 does not detect when the steps of removing storage box from the refrigerator or the like, refilling pump cylinder 125 and replacing to the refrigerator or the like are carried out. Therefore, control unit 210 transmits, upon pump cylinder 125 being refilled, a corresponding signal to temperature controller 410 via communication interface 407. In step S7, the reception of such a signal is checked. As long as no such signal is received, operational flow continues with step S5.

If ambient pressure sensor 422 is present, detection of a steep ambient temperature increase may be used by temperature controller 410 to automatically detect removal of storage box 400 from the refrigerator or the like.

In a further variant, an additional base station is provided that is designed to substantially permanently remain in the refrigerator or the like, even if storage box 400 is removed. Storage box 400 may be designed to operatively and optionally physically couple to the base station. Operative coupling may, e.g. be achieved by galvanic coupling via contacts, IFID-technology, short range inductive or capacitive coupling, or the like. Temperature controller 410 may detect the presence or absence of the operative coupling to the base station in order to decide whether storage box 400 is located inside or outside the refrigerator or the like. Optionally, the base station may include further components and functionality, for example a power supply for storage box 400.

If a signal indicative of an execution of pump cylinder refilling is received, operational flow continues with step S1 where the heating element S1 is switched off again.

Optionally, temperature controller 410 may, in waiting step S1, monitor the temperature of container 300 via temperature sensor 420. In case of the temperature rising above a pre-set threshold level and/or a temperature trend indicating an exceptional variation, an alerting signal may be transmitted to control unit 210 via communication interface 407 for providing a user alert.

Alternatively or additionally, an alerting device, such as an acoustic buzzer, may be included in container storage box 400. Situations where such an alarm may be provided are given, e.g., if it is forgotten to put container storage box 400 back to the refrigerator or the like, in case of a defective refrigerator, or in case of the refrigerator being mistakenly set to an unsuited temperature. For safety purposes, the temperature of container 300 may also be monitored in step S5.

Also optionally, the time interval may be monitored by temperature controller 410 and/or control unit 210 between starting warming step S5 and returning to waiting step S1. This time interval exceeding a pre-set threshold may be indicative of the user having forgotten to actually refill pump cylinder 125. Such time monitoring may be carried out additionally to monitoring the remaining filling level of pump cylinder 125.

The operational flow of Fig. 5 is also applicable for the operation of an alternative embodiments of a container storage box 400 that is designed to be generally kept at room temperature, with element 415 being a heating and cooling element, such as a Pelletier element. In such an embodiment, temperature controller 410 controls element 415, each in step S1 and S5, respectively, to maintain container 300 at a pre-set temperature, with container 300 being cooled to and subsequently maintained at a low storage temperature and container 300 being warmed to and subsequently maintained at a higher temperature, e.g., room temperature which the drug should have for refilling of pump cylinder 125.

In the embodiments of an ambulatory infusion system as shown in the Fig. 2, 3 and discussed so far, control unit 210 is shown as integral part of base unit 200. In such an embodiment, control unit 210 includes the user interface for operating the infusion system and optionally container storage box 400 and may in particular include a display, such as an LCD display or a touch screen, switches, pushbuttons, and the like. In alternative embodiments, control unit 210 is distributed and includes a base control unit that is integral part of base unit 200 and a further remote control unit as a separate device, with the base control unit and the remote control unit, in combination, forming control unit 210. The base control unit and the remote control unit favourable couple via a wireless RF connection, e.g. according to the Bluetooth® standard or the like.

In such an architecture, especially the user interface may be part of the remote control unit, e.g. in form of a handheld device. Such an embodiment is especially favourable in the context of infant therapy since a user interface is not required and may even be dangerous on those components that are carried by the infant. In a variant, however, the user interface is distributed between base control unit and remote control unit or is provided in a fully or partly redundant way on both separate devices.

In case of an ambulatory infusion system that is intended for infant therapy and includes a remote control unit, the communication range is favourably sufficient to enable communication within a house, potentially including locations such as garden or cellar.

If the remote control unit is a dedicated device, it may generally be designed and include functionality similar to the ACCU-CHEK® Aviva Combo Device that is designed for controlling an ACCU-CHEK® Spirit Combo insulin pump, both being available from Roche Diagnostics. In addition to controlling the infusion, the device may especially include a glucose measurement device, bolus advice functionality, and the like. The remote control device may further serve as "relay" to couple the ambulatory infusion systems to devices such as a PC, a cell phone, a continuous glucose monitor and a home network.

## Claims

1. Dosing unit (100) for an ambulatory infusion system (100, 200), the ambulatory infusion system (100, 200) being designed for the infusion of a liquid drug into a patient's body over an extended time period, the dosing unit (100) including

   a) an inlet port (105),
   b) an outlet port (115), the outlet port (115) being configured to couple to the patient's body (900), and a pump kernel, the pump kernel including
   c) a kernel body (125), the kernel body (125) forming a limiting surface of a stepwise or continuously variable dosing volume,
   d) a control valve (120), the control valve (120) being configured to couple the variable dosing volume alternatively, in a filling state (1 20a), to the inlet port (105) for filling or refilling the dosing unit(100) by increasing the dosing volume, or, in a infusing state (120b), to the outlet port (115) for dosing liquid drug out off the dosing unit (100) by deceasing the dosing volume, the control valve (120) sealing closing the inlet port (105) when the outlet port (115) is coupled to the dosing volume and sealing closing the outlet port (115) when the inlet port (105) is coupled to the dosing volume

   **characterized in that**
   the inlet port (105) is configured for being repeatedly manually attached to and detached from an external liquid drug container (300) and includes at least one of a self-sealing piercable septum, a self-closing fluidic coupler, and a check-valve, thus enabling a repeated sequence of attaching the external liquid drug container (300) to the inlet port (105), filling the dosing unit (100) with the control valve (120) being in the filling state (120a), detaching the liquid drug container (300) from the inlet port (105) and infusing drug from the dosing unit (100) with the control valve (120) being in the infusing state.

2. Dosing unit (100) according to either of the preceding claims, **characterized in that** the dosing unit (100) is designed to be continuously coupled, via the outlet port (115) to the patient's body (900) while filling the dosing unit (100).

3. Dosing unit (100) according to either of the preceding claims, **characterized in that** the dosing unit (100) is designed for fluidic decoupling the outlet port (115) from the patient's body prior to filling the dosing unit (100) and re-establishing the fluidic coupling to the patient's body (900) after the filling.

4. Dosing unit (100) according to either of the preceding claims, **characterized in that** the dosing unit (100) has a maximum filling in a range of 8 to 15 IU (International Units) of liquid insulin.

5. Dosing unit (100) according to either of the preceding claims, **characterized in that** the control valve (120) is designed as rotary valve or slide valve.

6. Dosing unit (100) according to claim 5, **characterized in that** the control valve (120) includes the kernel body (125) as a movable valve member and a further stationary valve member, the control valve (120) being switchable between the filling state (120a) and the infusing state (120b) by moving the kernel body (125) with respect to the stationary valve member.

7. Dosing unit (100) according to either of the preceding claims, **characterized in that** the pump kernel is a piston pump, with the kernel body (125) being a pump cylinder (125) and the pump kernel further including a piston (130), the piston (130) being displaceable, in particular linearly displaceable, arranged in a cavity of the pump cylinder (125), such that the surfaces of the cavity and the piston (130), in combination, define the dosing volume.

8. Dosing unit (100) according to either of the preceding claims, **characterized in that** the pump kernel includes a drive coupler for releasable coupling to a drive unit (215) with a single drive, the dosing unit (100) being designed to control force and/or torque transmission such that - during application - operation of the single drive alternatively effects both varying the dosing volume and switching the control valve (120) between the filling state (120a) and the infusing state (120b).

9. Dosing unit (100) according to either of claim 1 to claim 7, **characterized in that** the pump kernel includes a drive coupler for releasable coupling to a drive unit (215) with a dosing drive for varying the dosing volume and a valve drive for switching the control valve between the filling state and the infusing state, the valve drive being separate from the dosing drive.

10. Dosing unit (100) according to either of the preceding claims, **characterized in that** the dosing unit (100) includes a user-operable cradle-coupler, the cradle coupler being designed to engage a dosing unit coupler of a separate skin-mountable cradle, thus enabling attachment of the dosing unit (100) to the patient's body (900) via the cradle.

11. Ambulatory infusion system (100, 200), the ambulatory infusion system (100, 200) including

a) a dosing unit (100) according to either of the preceding claims,
b) a base unit (200), the base unit (200) including

• a drive unit (21 5), the drive unit (21 5) being designed to releasable couple to the dosing unit (100),
• an electronic control unit (210), the control unit (210) being coupled to the drive unit (215) for controlling operation of the infusion system alternatively in a filling mode for filling, by increasing the dosing volume, the dosing unit (100) with liquid drug from the liquid drug container (300), and in an infusing mode for infusing - by decreasing the dosing volume - liquid drug into the patient's body (900).

12. Ambulatory infusion system (100, 200) according to claim 11, **characterized in that** the control unit (210) is further designed to activate an alarming device when, during operation of the ambulatory infusion system (100, 200) in the infusing mode, a filling state of the dosing kernel approaches emptiness.

13. Ambulatory infusion system (100, 200) according to either of claim 11 or claim 12, **characterized in that** the control unit (210) is further designed, before switching the ambulatory infusion system (100, 200) to the infusing mode, to request a user confirmation input.

14. Ambulatory infusion system (100, 200) according to either of claim 11 to claim 13, **characterized in that** the control unit (210) is designed to operate the drive unit (215) to switch the valve arrangement from the infusing state into the filling state prior to increasing the dosing volume in the filling mode and from the filling state to the infusing state prior to decreasing the dosing volume in the dosing mode.

15. Ambulatory infusion system (100, 200) according to either of claim 11 to claim 14, **characterized in that** base unit (200) includes a coupling detector, the coupling detector being coupled to the control unit (210) to provide a signal indicating whether or not a drug container (300) is coupled to the inlet port (105).

16. Ambulatory infusion system (100, 200, 400) according to either of claim 11 to claim 15, **characterized in that** the ambulatory infusion system (100, 200, 400) further includes a container storage box (400), the container storage box (400) being a self-contained unit, the container storage box (400) including:

> a) a liquid drug container (300) or a compartment (405) for replaceable receiving a liquid drug container (300), the drug container (300) or the container compartment (405) being designed such that a fluidic outlet of the drug container can be coupled with the inlet port (105) without removing the drug container (300) from the storage box (400),
> b) a temperature control unit (410, 415, 420), the temperature control unit (410, 415, 420) being designed to vary a temperature of liquid drug that is stored inside the liquid drug container (300) in accordance with a time-varying schedule by actuating an electrically driven heating and/or cooling element (41 5).

17. Ambulatory infusion system (100, 200, 400) according to claim 16, **characterized in that** the temperature control unit (100, 200, 400) is configured to control the temperature of the liquid drug container (300) such that the temperature of liquid drug that is stored inside the liquid drug container (300) increases at instances at time that are correlated to instances of time for filling or refilling the dosing unit (100).

18. Ambulatory infusion system (100, 200, 400) according to either of claim 16 or claim 17, **characterized in that** the temperature control unit (410, 415, 420) includes at least one of

> - a clock relay, the clock relay controlling the heating and/or cooling element, or
> - a receiver, the receiver being configured to operatively couple to the control unit (210) and to receive from the control unit (210) a temperature control signal for varying the temperature of liquid drug inside the liquid drug container (300).

19. Ambulatory infusion system (100, 200, 400) according to either of claim 16 to claim 18, **characterized in that** the container storage box (400) is designed to be generally stored, during application, in a refrigerator.

20. Ambulatory infusion system (100, 200, 400) according to either of claim 11 to claim 19, further including a skin-mountable cradle, the cradle being separate from the dosing unit (100), wherein at least one of the dosing unit (100) and the base unit (200) includes a user-operable cradle coupler and the cradle includes a user-operable device coupler, the cradle coupler and the device coupler being designed for mutual engagement, thus enabling attachment of the dosing unit (100) to the patient's body (900) via the cradle.

21. Ambulatory infusion system (100, 200, 400) according to claim 20, **characterized in that** the device coupler and the cradle coupler are designed for a repeated sequence of engagement and disengagement.

**Patentansprüche**

1. Dosiereinheit (100) für ein ambulantes Infusionssystem (100, 200), wobei das ambulante Infusionssystem (100, 200) für die Infusion eines flüssigen Medikaments in einen Körper eines Patienten über eine längere Zeitdauer ausgelegt ist, wobei die Dosiereinheit (100) umfasst:

> a) eine Einlassöffnung (105),
> b) eine Auslassöffnung (115), wobei die Auslassöffnung (115) dazu konfiguriert ist, um mit dem Körper (900) des Patienten verbunden zu werden, und einen Pumpkern, wobei der Pumpkern umfasst:
> c) einen Kernkörper (125), wobei der Kernkörper (125) eine Grenzfläche von einem schrittweise oder kontinuierlich veränderbaren Dosiervolumen bildet,
> d) ein Steuerventil (120), wobei das Steuerventil (120) dazu konfiguriert ist, um das veränderbare Dosiervolumen alternativ in einem Befüllzustand (120a) mit der Einlassöffnung (105) zum Befüllen oder Wiederbefüllen der Dosiereinheit (100) durch Vergrößern des Dosiervolumens oder in einem Infusionszustand (120b) mit der Auslassöffnung (115) zum Dosieren flüssigen Medikaments aus der Dosiereinheit (100) durch Verringern des Dosiervolumens zu koppeln, wobei das Steuerventil (120) die Einlassöffnung (105) abdichtend verschließt, wenn die Auslassöffnung (115) mit dem Dosiervolumen verbunden ist, und die Auslassöffnung (115) abdichtend verschließt, wenn die Einlassöffnung (105) mit dem Dosiervolumen gekoppelt ist,

**dadurch gekennzeichnet, dass**
die Einlassöffnung (105) dazu konfiguriert ist, um wiederholt manuell an einem externen Flüssigmedikamentenbehälter (300) befestigt und davon gelöst zu werden, und zumindest eines von einem selbstdichtenden durchstechbaren Septum, einem selbstschließenden fluidischen Koppler und einem Absperrventil umfasst, wodurch eine wiederholte Abfolge von einem Befestigen des externen Flüssigmedikamentenbehälters (300) an der Einlassöffnung (105), einem Befüllen der Dosiereinheit (100), wobei sich das Steuerventil (120) im Befüllzustand (120a) befindet, einem Lösen des Flüssigmedikamentenbehälters (300) von der Einlassöffnung (105) und einem Infundieren eines Medikaments aus der Dosiereinheit (100), wobei sich das Steuerventil (120) im Infusionszustand befindet, ermöglicht wird.

2. Dosiereinheit (100) nach einem der vorstehenden Ansprüche, **dadurch gekennzeichnet, dass** die Dosiereinheit (100) dazu ausgelegt ist, um während einem Befüllen der Dosiereinheit (100) über die Auslassöffnung (115) kontinuierlich mit dem Körper (900) des Patienten gekoppelt zu sein.

3. Dosiereinheit (100) nach einem der vorstehenden Ansprüche, **dadurch gekennzeichnet, dass** die Dosiereinheit (100) dazu ausgelegt ist, um die Auslassöffnung (115) vor einem Befüllen der Dosiereinheit (100) fluidisch von dem Körper des Patienten zu entkoppeln, und nach dem Befüllen die fluidische Kopplung mit dem Körper (900) des Patienten. erneut herzustellen

4. Dosiereinheit (100) nach einem der vorstehenden Ansprüche, **dadurch gekennzeichnet, dass** die Dosiereinheit (100) eine maximale Befüllung in einem Bereich von 8 bis 15 IU (internationale Einheiten) von flüssigem Insulin hat.

5. Dosiereinheit (100) nach einem der vorstehenden Ansprüche, **dadurch gekennzeichnet, dass** das Steuerventil (120) als Drehventil oder Schieberventil designt ist.

6. Dosiereinheit (100) nach Anspruch 5, **dadurch gekennzeichnet, dass** das Steuerventil (120) den Kernkörper (125) als ein bewegliches Ventilelement und ein weiteres stationäres Ventilelement umfasst, wobei das Steuerventil (120) durch Bewegen des Kernkörpers (125) in Bezug auf das stationäre Ventilelement zwischen dem Befüllzustand (120a) und dem Infusionszustand (120b) umschaltbar ist.

7. Dosiereinheit (100) nach einem der vorstehenden Ansprüche, **dadurch gekennzeichnet, dass** der Pumpkern eine Kolbenpumpe ist, wobei der Kernkörper (125) ein Pumpzylinder (125) ist und der Pumpkern weiter einen Kolben (130) umfasst, wobei der Kolben (130) verschiebbar, insbesondere linear verschiebbar, in einem Hohlraum des Pumpzylinders (125) angeordnet ist, so dass die Oberflächen des Hohlraums und der Kolben (130) in Kombination das Dosiervolumen definieren.

8. Dosiereinheit (100) nach einem der vorstehenden Ansprüche, **dadurch gekennzeichnet, dass** der Pumpkern einen Antriebskoppler für eine lösbare Kopplung mit einer Antriebseinheit (215) mit einem einzigen Antrieb umfasst, wobei die Dosiereinheit (100) dazu designt ist, um eine Kraft- und/oder Drehmomentübertragung zu steuern, so dass während einer Anwendung ein Betrieb des einzigen Antriebs alternativ sowohl ein Verändern des Dosiervolumens als auch ein Umschalten des Steuerventils (120) zwischen dem Befüllzustand (120a) und dem Infusionszustand (120b) bewirkt.

9. Dosiereinheit (100) nach einem von Anspruch 1 bis Anspruch 7, **dadurch gekennzeichnet, dass** der Pumpkern einen Antriebskoppler zum lösbaren Koppeln mit einer Antriebseinheit (215) mit einem Dosierantrieb zum Verändern des Dosiervolumens und einem Ventilantrieb zum Umschalten des Steuerventils zwischen dem Befüllzustand und dem Infusionszustand umfasst, wobei der Ventilantrieb separat von dem Dosierantrieb ist.

10. Dosiereinheit (100) nach einem der vorstehenden Ansprüche, **dadurch gekennzeichnet, dass** die Dosiereinheit (100) einen durch einen Benutzer bedienbaren Halterungs-Koppler umfasst, wobei der Halterungs-Koppler dazu ausgelegt ist, um mit einem Dosiereinheit-Koppler einer separaten an der Haut montierbaren Halterung in Eingriff zu kommen, um dadurch eine Befestigung der Dosiereinheit (100) an dem Körper (900) des Patienten über die Halterung zu ermöglichen.

11. Ambulantes Infusionssystem (100, 200), wobei das ambulante Infusionssystem (100, 200) umfasst:

   a) eine Dosiereinheit (100) nach einem der vorstehenden Ansprüche,
   b) eine Basiseinheit (200), wobei die Basiseinheit (200) umfasst:

      - eine Antriebseinheit (215), wobei die Antriebseinheit (215) dazu ausgelegt ist, um lösbar mit der Dosiereinheit (100) zu koppeln,
      - eine elektronische Steuereinheit (210), wobei die Steuereinheit (210) mit der Antriebseinheit (215) gekoppelt ist, um einen Betrieb des Infusionssystems alternativ in einem Befüllmodus zum Befüllen der Dosiereinheit (100) mit einem flüssigen Medikament aus dem Flüssigmedikamentenbehälter (300) durch Vergrößern des Dosiervolumens, und in einem Infusionsmodus zum Infundieren - durch Verringern des Dosiervolumens - eines flüssigen Medikamentes in den Körper (900) des Patienten zu steuern.

**12.** Ambulantes Infusionssystem (100, 200) nach Anspruch 11, **dadurch gekennzeichnet, dass** die Steuereinheit (210) weiter dazu ausgelegt ist, eine Alarmvorrichtung zu aktivieren, wenn sich während eines Betriebs des ambulanten Infusionssystems (100, 200) in dem Infusionsmodus ein Befüllzustand des Dosierkerns dem Leerzustand annähert.

**13.** Ambulantes Infusionssystem (100, 200) nach Anspruch 11 oder Anspruch 12, **dadurch gekennzeichnet, dass** die Steuereinheit (210) weiter dazu ausgelegt ist, um vor einem Umschalten des ambulanten Infusionssystems (100, 200) in den Infusionsmodus eine Benutzerbestätigungseingabe anzufragen.

**14.** Ambulantes Infusionssystem (100, 200) nach einem von Anspruch 11 bis Anspruch 13, **dadurch gekennzeichnet, dass** die Steuereinheit (210) dazu designt ist, um die Antriebseinheit (215) zu betreiben, um die Ventilanordnung vor einem Vergrößern des Dosiervolumens in dem Befüllmodus von dem Infusionszustand in den Befüllzustand und vor einem Verringern des Dosiervolumens in dem Dosiermodus von dem Infusionszustand in den Dosiermodus umzuschalten.

**15.** Ambulantes Infusionssystem (100, 200) nach einem von Anspruch 11 bis Anspruch 14, **dadurch gekennzeichnet, dass** die Basiseinheit (200) einen Kopplungsdetektor umfasst, wobei der Kopplungsdetektor mit der Steuereinheit (210) gekoppelt ist, um ein Signal bereitzustellen, das angibt, ob ein Medikamentenbehälter (300) mit der Einlassöffnung (105) gekoppelt ist oder nicht.

**16.** Ambulantes Infusionssystem (100, 200, 400) nach einem von Anspruch 11 bis Anspruch 15, **dadurch gekennzeichnet, dass** das ambulante Infusionssystem (100, 200, 400) weiter eine Behälteraufbewahrungsbox (400) umfasst, wobei die Behälteraufbewahrungsbox (400) eine abgeschlossene Einheit ist, wobei die Behälteraufbewahrungsbox (400) umfasst:

a) einen Flüssigmedikamentenbehälter (300) oder ein Fach (405) zum austauschbaren Empfangen eines Flüssigmedikamentenbehälters (300), wobei der Medikamentenbehälter (300) oder das Behälterfach (405) designt ist, so dass ein fluidischer Auslass des Medikamentenbehälters mit der Einlassöffnung (105) gekoppelt werden kann, ohne den Medikamentenbehälter (300) aus der Aufbewahrungsbox (400) zu entfernen,

b) eine Temperatursteuereinheit (410, 415, 420), wobei die Temperatursteuereinheit (410, 415, 420) dazu designt ist, um eine Temperatur von einem flüssigen Medikament, das in dem Flüssigmedikamentenbehälter (300) aufbewahrt wird, in Übereinstimmung mit einem zeitveränderlichen Plan durch Betätigen eines elektrisch angetriebenen Heiz- und/oder Kühlelements (415) zu verändern.

**17.** Ambulantes Infusionssystem (100, 200, 400) nach Anspruch 16, **dadurch gekennzeichnet, dass** die Temperatursteuereinheit (100, 200, 400) dazu konfiguriert ist, um die Temperatur von dem Flüssigmedikamentenbehälter (300) zu steuern, so dass sich die Temperatur von einem flüssigen Medikament, das in dem Flüssigmedikamentenbehälter (300) aufbewahrt wird, zu Zeitpunkten erhöht, die mit Zeitpunkten zum Befüllen oder Wiederbefüllen der Dosiereinheit (100) korreliert sind.

**18.** Ambulantes Infusionssystem (100, 200, 400) nach einem von Anspruch 16 bis Anspruch 17, **dadurch gekennzeichnet, dass** die Temperatursteuereinheit (410, 415, 420) zumindest eines von den Folgenden umfasst:

- ein Takt-Relais, wobei das Takt-Relais das Heiz- und/oder Kühlelement steuert, oder
- einen Empfänger, wobei der Empfänger dazu konfiguriert ist, um sich betriebsmäßig mit der Steuereinheit (210) zu koppeln und von der Steuereinheit (210) ein Temperatursteuersignal zum Verändern der Temperatur eines flüssigen Medikaments in dem Flüssigmedikamentenbehälter (300) zu empfangen.

**19.** Ambulantes Infusionssystem (100, 200, 400) nach einem von Anspruch 16 bis Anspruch 18, **dadurch gekennzeichnet, dass** die Behälteraufbewahrungsbox (400) dazu designt ist, um während einer Anwendung im Allgemeinen in einem Kühlschrank aufbewahrt zu werden.

**20.** Ambulantes Infusionssystem (100, 200, 400) nach einem von Anspruch 11 bis Anspruch 19, weiter eine an der Haut montierbare Halterung umfassend, wobei die Halterung separat von der Dosiereinheit (100) ist, wobei zumindest eine von der Dosiereinheit (100) und der Basiseinheit (200) einen durch einen Benutzer betätigbaren Halterungskoppler umfasst und die Halterung einen durch einen Benutzer betätigbaren Vorrichtungskoppler umfasst, wobei der Halterungskoppler und der Vorrichtungskoppler für einen wechselseitigen Eingriff designt sind, wodurch eine Befestigung der Dosiereinheit (100) über die Halterung an dem Körper (900) des Patienten ermöglicht wird.

21. Ambulantes Infusionssystem (100, 200,400) nach Anspruch 20, **dadurch gekennzeichnet, dass** der Vorrichtungskoppler und der Halterungskoppler für einen wiederholte Abfolge von einem Eingriff und einem Lösen designt sind.

**Revendications**

1. Unité de dosage (100) pour un système de perfusion ambulatoire (100, 200), le système de perfusion ambulatoire (100, 200) étant conçu pour la perfusion d'un médicament liquide dans le corps d'un patient sur une période de temps prolongée, l'unité de dosage (100) comprenant :

    a) un orifice d'entrée (105),
    b) un orifice de sortie (115), l'orifice de sortie (115) étant configuré pour se coupler au corps (900) du patient, et
    un noyau de pompe, le noyau de pompe comprenant :
    c) un corps de noyau (125), le corps de noyau (125) formant une surface limite d'un volume de dosage variable par paliers ou en continu,
    d) une vanne de commande (120), la vanne de commande (120) étant configurée pour coupler le volume de dosage variable en alternance, dans un état de remplissage (120a), à l'orifice d'entrée (105) pour remplir ou recharger l'unité de dosage (100) en augmentant le volume de dosage, ou, dans un état de perfusion (120b), à l'orifice de sortie (115) pour doser le médicament liquide déchargé par l'unité de dosage (100) en réduisant le volume de dosage, la vanne de commande (120) scellant la fermeture de l'orifice d'entrée (105) lorsque l'orifice de sortie (115) est couplé au volume de dosage et scellant la fermeture de l'orifice de sortie (115) lorsque l'orifice d'entrée (105) est couplé au volume de dosage,

    **caractérisée en ce que :**

    l'orifice d'entrée (105) est configuré pour être manuellement fixé et détaché de manière répétée d'un récipient de médicament liquide externe (300) et comprend au moins l'un(e) d'un septum perçable auto-scellant, d'un coupleur fluidique auto-fermable et d'une vanne de retenue, ce qui permet une séquence répétée de fixation du récipient de médicament liquide externe (300) à l'orifice d'entrée (105), de remplissage de l'unité de dosage (100) avec la vanne de commande (120) à l'état de remplissage (120a), de détachement du récipient de médicament liquide (300) de l'orifice d'entrée (105) et de perfusion du médicament venant de l'unité de dosage (100) avec la vanne de commande (120) à l'état de perfusion.

2. Unité de dosage (100) selon l'une ou l'autre des revendications précédentes, **caractérisée en ce que** l'unité de dosage (100) est conçue pour être couplée en continu via l'orifice de sortie (115) avec le corps (900) du patient tout en remplissant l'unité de dosage (100).

3. Unité de dosage (100) selon l'une ou l'autre des revendications précédentes, **caractérisée en ce que** l'unité de dosage (100) est conçue pour un désaccouplage fluidique de l'orifice de sortie (115) du corps du patient avant remplissage de l'unité de dosage (100) et un rétablissement du couplage fluidique avec le corps (900) du patient après remplissage.

4. Unité de dosage (100) selon l'une ou l'autre des revendications précédentes, **caractérisée en ce que** l'unité de dosage (100) a un remplissage maximal dans une plage de 8 à 15 IU (International Units) d'insuline liquide.

5. Unité de dosage (100) selon l'une ou l'autre des revendications précédentes, **caractérisée en ce que** la vanne de commande (120) est conçue sous la forme d'une vanne rotative ou d'un robinet-vanne.

6. Unité de dosage (100) selon la revendication 5, **caractérisée en ce que** la vanne de commande (120) comprend le corps de noyau (125) comme élément de vanne mobile et un autre élément de vanne stationnaire, la vanne de commande (120) pouvant être commutée entre l'état de remplissage (120a) et l'état de perfusion (120b) en déplaçant le corps de noyau (125) par rapport à l'élément de vanne stationnaire.

7. Unité de dosage (100) selon l'une ou l'autre des revendications précédentes, **caractérisée en ce que** le noyau de pompe est une pompe à piston, le corps de noyau (125) étant un cylindre de pompe (125) et le noyau de pompe comprenant en outre un piston (130), le piston (130) pouvant être déplacé, en particulier déplacé de manière linéaire, agencé dans une cavité du cylindre de pompe (125) de sorte que les surfaces de la cavité et du piston (130) définissent en combinaison le volume de dosage ;

8. Unité de dosage (100) selon l'une ou l'autre des revendications précédentes, **caractérisée en ce que** le noyau de pompe comprend un coupleur d'entraînement pour se coupler de manière libérable à une unité d'entraînement (215) à entraînement unique, l'unité de dosage (100) étant conçue pour commander une transmission de force et/ou de couple de sorte que - au cours de l'application - le fonctionnement de l'entraînement unique affecte en alternance à la fois la variation du volume de dosage et la commutation de la vanne de commande (120) entre l'état de remplissage (120a) et l'état de perfusion (120b).

9. Unité de dosage (100) selon l'une ou l'autre de la revendication 1 à la revendication 7, **caractérisée en ce que** le noyau de pompe comprend un coupleur d'entraînement pour se coupler de manière amovible à une unité d'entraînement (215) avec un entraînement de dosage pour faire varier le volume de dosage et un entraînement de vanne pour commuter la vanne de commande entre l'état de remplissage et l'état de perfusion, l'entraînement de vanne étant séparé de l'entraînement de dosage.

10. Unité de dosage (100) selon l'une ou l'autre des revendications précédentes, **caractérisée en ce que** l'unité de dosage (100) comprend un coupleur à arceau actionnable par l'utilisateur, le coupleur à arceau étant conçu pour s'engager sur un coupleur d'unité de dosage d'un arceau séparé montable sur la peau, permettant ainsi la fixation de l'unité de dosage (100) au corps (900) du patient via l'arceau.

11. Système de perfusion ambulatoire (100, 200), le système de perfusion ambulatoire (100, 200) comprenant :

a) une unité de dosage (100) selon l'une ou l'autre des revendications précédentes,
b) une unité de base (200), l'unité de base (200) comprenant :

• une unité d'entraînement (215), l'unité d'entraînement (215) étant conçue pour se coupler de manière libérable à l'unité de dosage (100),
• une unité de commande électronique (210), l'unité de commande (210) étant couplée à l'unité d'entraînement (215) pour commander le fonctionnement du système de perfusion en alternance en mode de remplissage pour le remplissage, en augmentant le volume de dosage, de l'unité de dosage (100) par un médicament liquide provenant du récipient de médicament liquide (300), et en mode de perfusion pour perfuser - en réduisant le volume de dosage - le médicament liquide dans le corps (900) du patient.

12. Système de perfusion ambulatoire (100, 200) selon la revendication 11, **caractérisé en ce que** l'unité de commande (210) est en outre conçue pour activer un dispositif d'alarme lorsque, au cours du fonctionnement du système de perfusion ambulatoire (100, 200) en mode de perfusion, un état de remplissage du noyau de dosage se rapproche de l'état vide.

13. Système de perfusion ambulatoire (100, 200) selon l'une ou l'autre de la revendication 11 ou de la revendication 12, **caractérisé en ce que** l'unité de commande (210) est en outre conçue, avant de commuter le système de perfusion ambulatoire (100, 200) en mode de perfusion, pour demander une entrée de confirmation de l'utilisateur.

14. Système de perfusion ambulatoire (100, 200) selon l'une ou l'autre de la revendication 11 à la revendication 13, **caractérisé en ce que** l'unité de commande (210) est conçue pour faire fonctionner l'unité d'entraînement (215) pour commuter l'agencement de vannes de l'état de perfusion à l'état de remplissage avant d'augmenter le volume de dosage en mode de remplissage et de l'état de remplissage à l'état de perfusion avant de réduire le volume de dosage en mode de dosage.

15. Système de perfusion ambulatoire (100, 200) selon l'une ou l'autre de la revendication 11 à la revendication 14, **caractérisé en ce que** l'unité de base (200) comprend un détecteur de couplage, le détecteur de couplage étant couplé à l'unité de commande (210) pour fournir un signal indiquant si un récipient de médicament (300) est ou non couplé à l'orifice d'entrée (105).

16. Système de perfusion ambulatoire (100, 200, 400) selon l'une ou l'autre de la revendication 11 à la revendication 15, **caractérisé en ce que** le système de perfusion ambulatoire (100, 200, 400) comprend en outre une boîte de stockage de récipient (400), la boîte de stockage de récipient (400) étant une unité autonome, la boîte de stockage de récipient (400) comprenant :

a) un récipient de médicament liquide (300) ou un compartiment (405) pour recevoir de manière remplaçable un récipient de médicament liquide (300), le récipient de médicament (300) ou le compartiment de récipient (405) étant conçu de sorte qu'une sortie fluidique du récipient de médicament puisse être couplée à l'orifice d'entrée (105) sans retirer le récipient de médicament (300) de la boîte de stockage (400),
b) une unité de commande de température (410, 415, 420), l'unité de commande de température (410, 415, 420) étant conçue pour faire varier une température du médicament liquide qui est stocké à l'intérieur du récipient de médicament liquide (300) en fonction d'un programme variant dans le temps par actionnement d'un élément de chauffage et/ou de refroidissement (415) à entraînement électrique.

17. Système de perfusion ambulatoire (100, 200, 400) selon la revendication 16, **caractérisé en ce que** l'unité de commande de température (100, 200, 400) est configurée pour commander la température du récipient de médicament liquide (300) de sorte que la température du médicament liquide qui est stocké dans le récipient de médicament liquide (300) augmente à intervalles de temps qui sont en corrélation avec des intervalles de temps de remplissage ou de recharge de l'unité de dosage (100).

18. Système de perfusion ambulatoire (100, 200, 400) selon l'une ou l'autre de la revendication 16 ou de la revendication 17, **caractérisé en ce que** l'unité de commande de température (410, 415, 420) comprend au moins l'une des suivantes :

  - un interrupteur horaire, l'interrupteur horaire commandant l'élément de chauffage et/ou de refroidissement, ou
  - un récepteur, le récepteur étant configuré pour se coupler en service à l'unité de commande (210) et recevoir de l'unité de commande (210) un signal de commande de température pour faire varier la température du médicament liquide à l'intérieur du récipient de médicament liquide (300).

19. Système de perfusion ambulatoire (100, 200, 400) selon l'une ou l'autre de la revendication 16 à la revendication 18, **caractérisé en ce que** la boîte de stockage de récipient (400) est conçue pour être stockée de manière générale au cours de l'application dans un réfrigérateur.

20. Système de perfusion ambulatoire (100, 200, 400) selon l'une ou l'autre de la revendication 11 à la revendication 19, comprenant en outre un arceau montable sur la peau, l'arceau étant séparé de l'unité de dosage (100), dans lequel au moins l'une de l'unité de dosage (100) et de l'unité de base (200) comprend un coupleur à arceau actionnable par l'utilisateur et l'arceau comprend un coupleur de dispositif actionnable par l'utilisateur, le coupleur à arceau et le coupleur de dispositif étant conçus pour un engagement mutuel, permettant ainsi la fixation de l'unité de dosage (100) au corps (900) du patient via l'arceau.

21. Système de perfusion ambulatoire (100, 200, 400) selon la revendication 20, **caractérisé en ce que** le coupleur de dispositif et le coupleur à arceau sont conçus pour une séquence répétée d'engagement et de dégagement.

**Figure 1 (prior art)**

**Figure 4**

**Figure 5**

**Figure 2**

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1970677 A1
- EP 2163273 A1
- EP 2361646 A1
- EP 2457602 A1
- WO 200906635 A2
- WO 2004110526 A1